(19) Europäisches Patentamt
European Patent Office
Office européen des brevets

(11) EP 1 484 413 A1

(12) **EUROPEAN PATENT APPLICATION**

(43) Date of publication:
**08.12.2004 Bulletin 2004/50**

(51) Int Cl.[7]: **C12Q 1/18**, C12Q 1/26, C12Q 1/28

(21) Application number: **03012155.2**

(22) Date of filing: **03.06.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Flohé, Leopold, Prof. Dr.**
**38124 Braunschweig (DE)**

(72) Inventors:
- **Jäger, Timo**
**38106 Braunschweig (DE)**
- **Flohé, Leopold, Prof. Dr.**
**38304 Wolfenbüttel (DE)**

(74) Representative: **Boeters, Hans Dietrich, Dr. et al**
**Patentanwälte Boeters & Bauer,**
**Bereiteranger 15**
**81541 München (DE)**

Remarks:
The sequence listing, which is published as annex to the application documents, was filed after the date of filing. The applicant has declared that it does not include matter which goes beyond the content of the application as filed.

(54) **Assay for identifying inhibitors of Mycobacterium anti-oxidant defense system**

(57) The invention relates to an enzyme-based assay for detecting inhibitors of the anti-oxidant defense system in *Mycobacterium* involving a thioredoxin reductase or an alkylhydroperoxidase, a method for manufacturing a pharmaceutically active compound for treating and/or preventing a *Mycobacterium* associated disease and uses thereof.

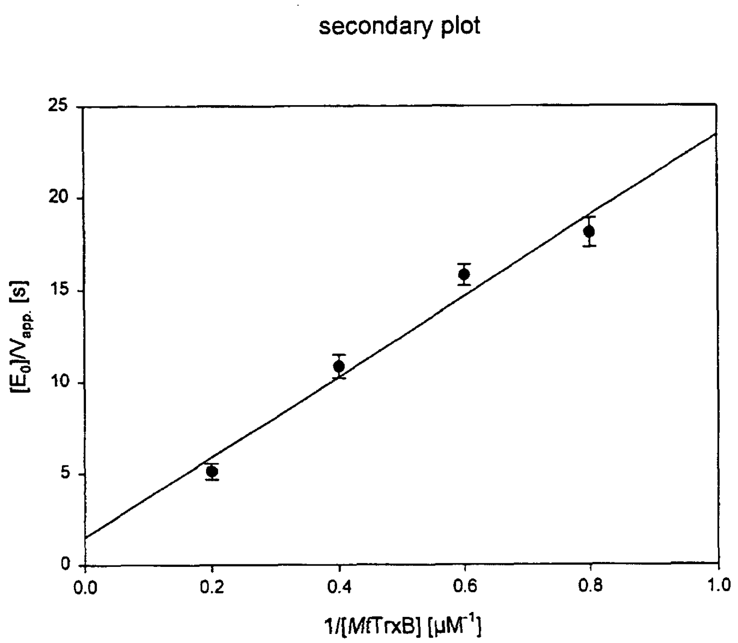


Figure 2B



EP 1 484 413 A1

## Description

### Field of the invention

The present invention pertains generally to an enzyme-based assay for detecting inhibitors of the anti-oxidant defense system in *Mycobacterium.* In a first aspect, the enzyme-based assay comprises a reductant, a thioredoxin reductase or a peroxidase, a donor substrate capable of receiving a hydrogen atom from said thioredoxin reductase or said peroxidase, and a hydroperoxide. The enzyme-based assay is capable for determining the consumption of the reductant.

Furthermore, the invention relates to a method for manufacturing a pharmaceutically active compound for the treatment and/or the prevention of a *Mycobacterium* associated disease. In a first aspect, the method comprises the steps of providing an enzyme-based assay for detecting inhibitors of the anti-oxidant defence system in Mycobacterium, providing a test sample comprising a potentially active compound, contacting the enzyme-based assay with the test sample, determining the consumption of a reductant and recovering the active compound.

Additionally, the invention refers to a pharmaceutically active compound, which is identified by the method for manufacturing said pharmaceutically active compound. Said compound is used for the preparation of a medicament for treating and/or preventing a *Mycobacterium* associated disease.

Several documents are cited throughout the text of this specification. Each of the documents cited herein (including any manufacture's specifications, instructions etc.) are hereby incorporated by reference. However, there is no admission that any document cited is indeed prior art to the present invention.

### Background of the invention

*Mycobacteria* cause two of the most important diseases in history, tuberculosis and leprosy. Even though the antibiotic streptomycin, which possesses a broad spectrum of activity, is usually active against *Mycobacterium tuberculosis,* many clinical isolates have been shown to be resistant. Further, resistance of *Mycobacterium tuberculosis* to the tuberculostatic isoniazid (INH) is detected in 4-8 % of clinical isolates in the United States and is much higher in other populations (Pablos-Mendez et al., (1998), *N. Engl. J. Med.* 338, 1641-1649). It is assumed that IHN resistance is at least in part due to a loss-of-function mutation of a key enzyme in the metabolic pathway.

Bacteria, as living organisms need energy to survive. This energy is produced by a process known as oxidative phosphorylation, in form of the chemical entity adenosine triphosphate (ATP). A major role in the production of ATP play two pyrimidine nucleotides, namely nicotinamide adenine dinucleotide (NAD) and nicotinamide adenine dinucleotide phosphate (NADP). These compounds mediate most often biological oxidations and reductions, i. e., they serve as acceptors for hydrogen atoms released by dehydrogenation reactions, and as donors of hydrogen atoms required for hydrogenation reactions. Both of these pyrimidine nucleotides can readily undergo reversible oxidation and reduction.

Bacteria do not only have to produce energy in order to survive, but they also have to deal with oxygen. All bacteria contain certain enzymes, which react with oxygen, e.g., superoxide dismutase, catalase and peroxidase. These enzymes protect the cell from the toxic consequences of oxygen metabolism, as they eliminate oxygen radicals and hydrogen peroxide ($H_2O_2$).

Organisms living under aerobic conditions have developed various anti-oxidative mechanisms to protect themselves from damage by reactive oxygen species. A family of anti-oxidative proteins, designated as peroxiredoxin, has been identified. They share a common reactive cysteine residue in the N-terminal region, and are capable of serving as a peroxidase and involve thioredoxin and/or glutathione as the electron donor.

Peroxiredoxins are low efficiency peroxidases using thiols as reductants. They appear to be fairly promiscuous with respect to the hydroperoxide substrate; the specificity for the donor substrate varies considerably between the subfamilies, comprising GSH, thioredoxin, tryparedoxin and the analogous CXXC motifs in bacterial AhpF proteins. Peroxiredoxins are responsible for anti-oxidant defense in bacteria (AhpC), yeast (thioredoxin peroxidase) and trypanosomatids (tryparedoxin peroxidase). They are considered to determine virulence of mycobacteria.

Thioredoxins are small molecular weight disulfide oxidoreductases specialized in the reduction of disulfide bonds on other proteins. Generally, the enzymes which are selectively and reversibly reduced by these proteins oscillate between an oxidized and inactive conformation and a reduced and active conformation. Thioredoxin constitutes the archetype of a family of protein disulfide oxidoreductase which comprises glutaredoxin and protein disulfide isomerase. Thioredoxin and glutaredoxin serve many roles in the cell, including the redox regulation of target enzymes and transcription factors. They can also serve as hydrogen donors to peroxiredoxins, the function of which is to get rid of hydroperoxide in the cell.

INH resistance of *Mycobacterium tuberculosis* is supposed to be due to a loss-of-function mutation of catalase (katG) (Sherman et al., (1996), *Science* 272, 1641-1643) an enzyme, which is required for the activation of INH (Zhang et al., (1992), *Nature* 358, 591-593), but appears equally important for removal of $H_2O_2$. Catalase-negative clinical isolates constitutively overexpress AhpC, which is a two-cysteine-peroxiredoxin-type peroxidase, whereas catalase containing strains (*kat*$G^-$ strains) generated *in vitro,* which do not overexpress AhpC, proved to be virulent. This revealed that the catalase deficiency had to be compensated by another peroxidase, such as AhpC, to assure protection of the pathogen against the oxidant attack of the host's phagocytes (Sherman et al., (1999), *Biofactors* 10, 211-217). However, it is unclear in the prior art how AhpC is supplied with reduction equivalents NAD(H) and/or NADP(H), since the flavoprotein AhpF, which complements the alkyl hydroperoxide reductase systems in most bacteria (Hofmann et al., (2002), *Biol. Chem.* 383, 347-364; Wood et al., (2003) *Trends Biochem. Sci.* 28, 32-40) is lacking in *Mycobacterium tuberculosis* (Cole et al., (1998), *Nature* 393, 537-544).

As stated above, many clinical isolates of *Mycobacterium* are resistant to commercially available antibiotics. This is not only a problem with respect to an adequate treatment of infected patients, who bear the risk of developing a chronic infection, but it is also a serious problem for test systems, which are currently used to identify strains and clinical isolates in samples. In order to develop new highly efficient antibiotic compounds, there is a need for an assay system, which is capable of identifying such active compounds.

**Summary of the invention**

The present invention relates to a novel enzyme-based assay for detecting inhibitors of the anti-oxidant defense system in *Mycobacterium.* Furthermore, the present invention relates to a method for manufacturing a pharmaceutically active compound for treating and/or preventing a *Mycobacterium* associated disease. The enzyme-based assay and the method for manufacturing a pharmaceutically active compound are useful for the identification of compounds active in the treatment and/or prevention of Mycobacterium associated diseases. With the help of such pharmaceutically active compounds, diseases like tuberculosis caused by antibiotic resistant strains, can be efficiently treated and/or prevented. Therefore, the invention additionally relates to the use of a pharmaceutically active compound identified by a method for manufacturing pharmaceutically active compounds for the preparation of a medicament for treating and/or preventing a *Mycobacterium* associated disease.

**Brief description of the drawings**

The disclosure of this invention may best be understood in conjunction with the accompanying drawings, in which

Figure 1 shows oxidative activation of *Mycobacterium tuberculosis* AhpC (*Mt*AhpC) and *Mycobacterium tuberculosis* AhpD (*Mt*AhpD) by t-butyl hydroperoxide (t-bOOH). *Mt*AhpC activity with *Mt*AhpD as cosubstrate was tested in the conventional test system.

Figure 2 shows a steady-state-kinetic analysis of *Mycobacterium* tuberculosis peroxiredoxin TPx (*Mt*TPx) with *Mycobacterium tuberculosis* thioredoxin B (*Mt*TrxB) or *Mycobacterium tuberculosis* thioredoxin C (*Mt*TrxC) as cosubstrates.

**Detailed description of the invention**

In view of the need of therapeutic means for the prevention and treatment of diseases related to mycobacteria, the technical problem underlying the invention is to provide means and methods for the identification of pharmaceutically active compounds, which act as inhibitors of the anti-oxidant defense system in *Mycobacterium.*

The solution to the technical problem is achieved by providing following embodiments of the present invention.

Accordingly, in a first aspect the invention relates to an enzyme-based assay for detecting inhibitors of the anti-oxidant defense system in *Mycobacterium* comprising

(i) a reductant;
(ii) a thioredoxin reductase or a peroxidase;
(iii) a donor substrate capable of receiving a hydrogen atom from (ii); and
(iv) a hydroperoxide,

for determining the consumption of the reductant.

In a preferred embodiment of the present invention, the thioredoxin reductase and/or the peroxidase and/or the donor substrate is an enzyme derived from *Mycobacterium.* A particularly preferred bacterium is *Mycobacterium tuberculosis.*

In another embodiment, the enzyme is expressed in heterologous host cells. Preferred host cells are cells of bacteria, yeasts, insect cells or mammalian cells. Particularly preferred are cells of *Escherichia coli, Saccharomyces cerevisiae* and *Spodoptera frugiperda.*

The reductant, which is used in the enzyme-based assay is preferably NADH, NADPH and/or FADH.

In another preferred embodiment of the present invention, the peroxidase, which is used in the enzyme-based assay, is a peroxiredoxin. Preferred are AhpC and/or TPx of *Mycobacterium,* and enzymes of *Mycobacterium tuberculosis* is most preferred.

The donor substrate, which is used in the enzyme-based assay is preferably glutathione or thioredoxin. Preferred are thioredoxin B (TrxB) and/or C (TrxC) of *Mycobacterium,* and enzymes of *Mycobacterium tuberculosis* are most preferred.

The hydroperoxide, which is used in the enzyme-based assay, can be hydrogen peroxide, t-butyl hydroperoxide, cumene hydroperoxide, linoleic acid hydroperoxide, phosphatidyl choline hydroperoxide or any oxidant comprising a hydroperoxy group.

In another aspect, the present invention relates to a method for manufacturing a pharmaceutically active compound for treating and/or preventing a *Mycobacterium* associated disease, comprising the steps of

(a) providing an enzyme-based assay comprising a reductant, a thioredoxin reductase or a peroxidase, a donor substrate capable of receiving a hydrogen atom from the thioredoxin reductase or the peroxidase, and a hydroperoxide;
(b) providing a test sample comprising a potentially active compound;
(c) contacting (a) and (b);
(d) determining consumption of a reductant;
(e) recovering the active compound.

In a preferred embodiment, the reductant used in the method for manufacturing a pharmaceutically active compound is NADH, NADPH and/or FADH.

In another embodiment, the active compound is combined with suitable pharmaceutically acceptable fillers, excipients and/or additives. Preferred are physiological saline solution, demineralised water, stabilizers, proteinase inhibitors and gel formulations.

In a third aspect, the present invention relates to the use of the pharmaceutically active compound identified by the method for manufacturing a pharmaceutically active compound, for the preparation of a medicament for treating and/or preventing a disease, which is associated with *Mycobacterium.*

In a preferred embodiment, the disease is tuberculosis, which is associated with *Mycobacterium tuberculosis.*

**Best method to carry out the invention**

Thioredoxin C that, by analogy to many eukaryotic systems, was suspected to reduce peroxiredoxins. However, it was reported to be inactive as potential alternative reductant of mycobacterial AhpC (Hillas et al., (2000), *J. Biol. Chem.* 275, 18801-18809), as was mycothiol, the major low molecular weight thiol of mycobacteria (Vergauwen et al., (2001), *Arch. Physiol. Biochem.* 113, B22).

Even though thioredoxin C was convincingly demonstrated to be inactive in reducing AhpC, the inventors screened the genome of *Mycobacterium tuberculosis* (Cole et al., (1998), *Nature* 393, 537-544) for homologues of AhpF, thioredoxin-related proteins and peroxiredoxins. Subsequently, they expressed the genes heterologously in *Escherichia coli* or *Mycobacterium smegmatis*, and were surprisingly able to reconstitute peroxidase systems from the isolated gene products *in vitro.* Further, the inventors checked the efficiencies of the heterologously expressed proteins by steady-state kinetics.

The inventors demonstrated that mycothiol reductase of (*Mt*MycR), which is a homologue of AhpF, does not reduce *Mt*AhpC. Neither did mycothiol if continuously regenerated by NAD(P)H via *Mt*MycR.

Further, the inventors demonstrated that *Mt*AhpD is reduced by liponamide and can in turn reduce *Mt*AhpC. Liponamide, kept reduced by NADH and bovine liponamide reductase, plus *Mt*AhpD and *Mt*AhpC constituted a peroxidase system. The time progression curves, however, were unusual in starting with a lag phase when the fully reduced system was started with t-bOOH as *Mt*AhpC substrate (Figure 1). Reversal inhibition by the initial high hydroperoxide concentration does not account for the phenomenon, since it is identically observed from 20 µM up to 73 µM initial *t*-bOOH.

Surprisingly, the reaction started at maximum rate if *Mt*AhpC and *Mt*AhpD were preincubated with *t*-bOOH for 2 min, indicating a facilitated interaction of the reaction partners. The x-ray structure of AhpD shows that the reactive CXXC motifs of AhpD are hidden in the interior of the trimeric protein (Bryk et al., (2002), *Science* 295, 1073-1077; Nunn et al., (2002), *J. Biol. Chem.* 277, 20033-20040), and *Mt*AhpC tends to form decameric rings in dependence of its redox status (Chauhan and Mande, (2001), *Biochem. J.* 354, 209-215; Wood et al., (2002), *Biochemistry* 41, 5493-5504). Redox-dependent conformational changes might therefore determine the accessibility of the *Mt*AhpD reaction centers for *Mt*AhpC. Steady-state-analysis of the apparently regular parts of the turnover curves yielded a net forward rate constant of *Mt*AhpC for the reaction with *t*-bOOH, $k'_1$, of 1 x $10^4$ $M^{-1}s^{-1}$, which marks the lower edge of corresponding peroxiredoxin constants (Hofmann et al., (2002), *supra*; Wood et al., (2003), supra), and a $k'_2$ for the reduction by *Mt*AhpD of 2.7 x $10^6$ $M^{-1}s^{-1}$ which, like the $K_{M\ AhpD}$ of 0.23 μM, clearly demonstrates specific reactivity.

Additionally, the inventors were surprisingly successful in reconstituting mycobacterial peroxidase systems *in vitro* by combining AhpC with thioredoxin reductase (*Mt*TR) and various CXXC-containing proteins. *Mt*TR did not reduce *Mt*AhpD, *Mt*TrxA and the glutaredoxin related Rv2466c gene product but only *Mt*TrxB and *Mt*TrxC. Surprisingly, *Mt*TrxC efficiently reduced *Mt*AhpC. The kinetic analysis of *Mt*AhpC in the system reconstituted by homologous TR and TrxC yielded a $k'_1$ of 2.3 x $10^4$ $M^{-1}s^{-1}$ that is nearly identical to that obtained in the AhpD system. Both the $k'_2$ and the $K_M$ characterizing the reactivity and/or affinity to TrxC compare unfavourably with the constants for AhpD by a factor of 10 and 20, respectively, as does the $k_{cat}$ (Table 1), yet the relative cellular abundancies of the potential AhpC reductants (see below) qualify *Mt*TrxC as the relevant one.

*Mt*TrxC also proved to reduce another mycobacterial peroxiredoxin (Rv1932) that by sequence homology, had been classified as a "thiol peroxidase" (TPx) but had never been investigated in respect to activity and specificity. *Mt*TPx also accepted *Mt*TrxB although with slightly lower affinity and reactivity (Table 1). *Mt*TPx belongs to a subfamily of two-cysteine-peroxiredoxins which, in contrast to AhpC and other two-cysteine-peroxiredoxins, are considered to be functionally monomeric. Their mechanism of action is believed to involve oxidation of an N-proximal cysteine residue, here C60, to a sulfenic acid derivative by the hydroperoxide substrate followed by formation of an intramolecular disulfide bridge with a distal cysteine, here C94, while in the oligomeric two-cys peroxiredoxins the corresponding reaction center is build up between the subunits (Hofmann et al., (2002), *supra;* Wood et al., (2003), *supra*; Baker and Poole, (2003), *J. Biol. Chem.* 278, 9203-9211).

Steady-state kinetics of *Mt*TPx with *t*-butyl hydroperoxide and *Mt*TrxB as substrates display a ping-pong pattern, as is typically observed with peroxidases (Figure 2A, B). With *Mt*TrxC as substrate, however, the slopes in reciprocal primary plots (Figure 2C) surprisingly appear not strictly parallel and show marked deviations from linearity at high peroxide concentrations that are particularly pronounced at low cosubstrate levels. This kinetic anomaly is also seen with AhpC. This phenomenon is interpreted as being indicative of negative cooperativity between subunits. Gel permeation of native *Mt*TPx yielded an average size of 36 kDa that underscores its homodimeric nature. The kinetic anomalies of MtTPx could thus be due to an allosteric phenomena.

Interestingly, the peroxidatic efficiency of *Mt*TPx is about one order of magnitude higher than that of *Mt*AhpC. With *Mt*TrxB as cosubstrate a $k'_1$ value 3.3 x $10^5$ $M^{-1}s^{-1}$ was measured that appears more reliable than the corresponding value obtained with *Mt*TrxC of 0.9 x $10^5$ $M^{-1}s^{-1}$. In a ping-pong mechanism, the values should be identical but the latter is likely underestimated due to the deviations from linearity of the reciprocal plots (Figure 2C). The $k'_2$ values of *Mt*TPx for both thioredoxins are similar and slightly higher than that of *Mt*AhpC for *Mt*TrxC but lower than that of the *Mt*AhpC/*Mt*AhpD couple. The highest $V_{max}$ is obtained for the *Mt*TPx/*Mt*TrxC system (Table 1).

The kinetic constants compiled in table 1 and the general rate equation for two-substrate ping-pong mechanisms

$$[E_o]/v = \Phi_o + \Phi_1/[ROOH] + \Phi_2/[reductant]$$

allow to estimate turnover rates by the different systems if, as a first approximation, the peculiarities of peroxiredoxin kinetics at extreme substrate concentrations are ignored. However, the concentrations of the enzymes appear to vary between strains and substrate concentrations can only be roughly estimated. At low peroxide tone and high supply of reductants, as is commonly assumed for unstressed situations, the turnover depends on the level of reduced peroxidase that should approximately equal $[E_0]$, on $k'_1$ and on the peroxide concentration.

$$v = k'_1\,[E_{red}]\,[ROOH]$$

AhpC is hardly detectable in most of the *kat*G$^+$ strains of *Mycobacterium tuberculosis* and *Mycobacterium marinum* while it is easily found in *Mycobacterium smegmatis* and *Mycobacterium bovis* BCG. In contrast, *Mt*TPx is consistently seen in proteomes of *Mycobacterium tuberculosis.* Its concentration may be estimated much higher than the concentration of AhpC. Being the dominant peroxiredoxin and having a higher $k'_1$ than AhpC, *Mt*TPx must definitely be rated as the more relevant enzyme under physiological conditions. Under $H_2O_2$ stress, the reductant capacity may become limiting according to

$$v = k'_2 [E_{ox}] [reductant]$$

The concentration of the reductants is linked in a complex manner to the NADH and NADPH pools, depends on the speed of regenerating enzymes and the pool size of the proximal reductants, only the latter being easily estimated. AhpD can be identified in *Mycobacterium tuberculosis* proteomes as a minor spot corresponding to a low molarity level. MtTrxB is characterized here as a specific substrate of *Mt*TPx, but MtTrxC is always seen as a major protein in all *Mycobacterium tuberculosis* strains investigated. *Mt*TR is a medium spot in all analyzed mycobacterial proteomes (Jungblut et al., (1999), *Mol. Microbiol.* 33, 1103-1117; Mollenkopf et al., (1999), *Electrophoresis* 20, 2172-2180; Nagai et al., (1991), *Infect. Immun.* 59, 372-382; Sonnenberg and Belisle, (1997), Infect. Immun. 65, 4515-4524 ;Weldingh et al., (1998), *Infect. Immun.* 66, 3492-3500 ; Rosenkrands et al., (2000), *Electrophoresis* 21, 935-948 ; Rosenkrands et al., (2000), *Electrophoresis* 21, 3740-3756. Thus, the *Mt*TR/TrxC-mediated detoxification systems, because of their high reduction capacities, are particularly relevant under conditions of oxidative stress.

Taking into account both rate constants and capacities, NADPH-driven $H_2O_2$ reduction by the *Mt*TR/*Mt*TrxC/*Mt*AhpC and the *Mt*TR/*Mt*TrxC/*Mt*TPx system should be at least as efficient as the NADH-driven, AhpD/AhpC-mediated one in coping with $H_2O_2$ challenge int *kat*G$^-$ strains. Both peroxiredoxin-type peroxidases may, however, meet special antioxidant requirements also in *kat*G$^+$ strains. Like other peroxiredoxins, the mycobacterial enzymes act on a broad spectrum of hydroperoxides, *Mt*TPx even on complex lipid hydroperoxides (Table 2). *Mt*AhpC and *Mt*TPx therefore complement catalase in antioxidant defense of Mycobacterium *tuberculosis* as do broad spectrum selenoperoxidases, such as GPx-4, in the host (Flohé and Brigelius-Flohé, (2001), in: *Selenium. Its Molecular Biology and Role in Human Health,* D. L. Hatfield, Ed. (Kluwer Academic Publishers, Boston/Dordrecht/London), and thereby contribute in general to the pathogen's resistance to the oxidative stress exerted by the innate immune response.

**Examples**

The invention will now be described in detail with respect to showing how certain specific representatives embodiments thereof can be implemented. However, all examples are intended to be illustrative only. In particular, the invention is not intended to be limited to method, materials, conditions, process parameters and the like specifically recited herein.

Example 1

Preparation of constructs

*Mt*MycR preparation

The MycR open reading frame (ORF) was amplified from *Mycobacterium tuberculosis* H37Rv genomic DNA by PCR with engineered 5' *Pst*I and 3' *Hin*dIII sites and a C-terminal 6xHis tag (MtMycR Forward: 5'-NNN NNN **CTG CAG** ATG GAA ACG TAC GAC ATC GCG ATC-3'; *Mt*MycRH6 Reverse: 5'-NNN **NNN AAG CTT** TCA GTG ATG GTG ATG GTG ATG ACG CAG GCC AAG CAG CGC GTT-3'). The PCR product was cloned into the mycobacterial expression vector pMV261. Protein expression was induced in *Mycobacterium smegmatis* by heat shock (45°C). The soluble protein was purified by nickel chelate chromatography (Ni-NTA Superflow; Qiagen, Hilden, Germany).

MycSH preparation

*Mycobacterium smegmatis* cells were disrupted by boiling them for 10 min at 95°C in 10 mM Tris-HCl pH 8. MycSH was isolated from the crude *Mycobacterium smegmatis* soluble extract essentially as described by Unson et al., (1998), J. *Immunol. Methods* 214, 29-39.

*Mt*AhpC preparation

The AhpC ORF was amplified by PCR using a forward primer that contained an NdeI site and overlapped the 5' end of the coding sequence (*Mt*AhpC Forward: 5'-G GCG CGC **CAT ATG** CCA CTG CTA ACC ATT GGC GA-3') and a reverse primer which contained a *Bam*HI site, the sequence for a 6xHis tag and overlapped the 3' end of the coding sequence (*Mt*AhpCH6 Reverse: 5'-CCG GCG **GGA TCC** TTA GTG GTG GTG GTG GTG GTG GGC CGA AGC CTT GAG GAG TT-3'). The PCR product was cloned into the procaryotic expression vector pET22b(+) (Novagen, Madison, USA) digested with corresponding sets of enzymes. Expression was induced in *Escherichia coli* Tuner (DE3) (Novagen, Madison, USA) with 0.5 mM IPTG at 30°C. The soluble protein was purified by nickel chelate chromatog-

raphy.

*Mt*AhpD preparation

The AhpD ORF was amplified by PCR using primers with engineered 5' *Nde*I and 3' HindIII sites (MtAhpD Forward: 5'-CC CCC CCC **CAT ATG** AGT ATA GAA AAG CTC AAG GCC GCG-3'; MtAhpD Reverse: 5'-CC CCC CCC **AAG CTT** TTA GCT TGG GCT TAG TGC CTC GAT-3') and cloned into pET22b(+). Expression was induced in *E. coli* Tuner (DE3) with 0.5 mM IPTG at 30°C. *Mt*AhpD was purified to homogeneity by anion exchange chromatography (UNOsphere Q; BioRad, Munich, Germany) and hydrophobic interaction chromatography (Phenyl Sepharose 6; Amersham Biosciences, Freiburg, Germany).

*Mt*TR preparation

The vector pTrcHis A (Invitrogen, Karlsruhe, Germany) containing the TR ORF fused to an N-terminal 6xHis tag was kindly provided by T.H.M. Ottenhoff (Leiden University Medical Centre, Leiden, The Netherlands). Protein expression and purification was done as described for MtAhpC.

*MtTrxA, MtTrxB* and *Mt*Rv2466c preparation

The TrxA, TrxB and Rv2466c ORFs were amplified by PCR using forward primers that contained an NdeI site and overlapped the 5' end of the coding sequence (MtTrxA Forward: 5'-CCC CCC **CAT ATG** GTG ACC ACT CGA GAC CTC ACG-3'; *Mt*TrxB Forward: 5'-CCC CCC **CAT ATG** GTG ACT ACC CGA GAC CTC ACT GCC-3'; *Mt*Rv2466c Forward: 5'-CCC CCC **CAT ATG** CTC GAG AAG GCC CCC CAG-3') and reverse primers which contained an *Eco*RI site, the sequence for a 6xHis tag and overlapped the 3' end of the coding sequence (*Mt*TrxAH6 Reverse: 5'-CCC CCC **GAA TTC** TCA GTG ATG GTG ATG GTG ATG GGA TGA AGT CTT TGT TCC AGG GCC-3'; *Mt*TrxBH6 Reverse: 5'-CCC CCC **GAA** TTC TCA GTG ATG GTG ATG GTG ATG GGC TTG TTG GGC TCG CCC GTT-3'; MtRv2466cH6 Reverse: 5'-CCC CCC **GAA TTC** CTA GTG GTG GTG GTG GTG GTG GTC GAA CTG AGG CGG CTC GGT-3'). The PCR products were cloned into pET22b(+) digested with corresponding set of enzymes.

Expression and purification was done as described for MtAhpC.

*Mt*TrxC and *Mt*TPx preparation

The TrxC and TPx ORFs were amplified by PCR using forward primers that contained an *Nde*I site, the sequence for a 6xHis tag and overlapped the 5' end of the coding sequence (*Mt*H6TrxC Forward: 5'-CC CCC CCC **CAT ATG** CAT CAC CAT CAC CAT CAC ACC GAT TCC GAG AAG TCC GCC-3'; *Mt*H6TPx Forward: 5'-CCC CCC **CAT ATG** CAT CAC CAT CAC CAT CAC GCA CAG ATA ACC CTG CGA GGA-3') and reverse primers which contained a *Hind*III site and overlapped the 3' end of the coding sequence (*Mt*TrxC Reverse: 5'-CC CCC CCC **AAG CTT** CTA GTT GAG GTT GGG AAC CAC GTC-3'; *Mt*TPx Reverse: 5'-CCC CCC **AAG CTT** CTA GGC GCC CAG CGC GGC-3'). The PCR products were cloned into cloned pET22b(+) digested with corresponding set of enzymes. Expression and purification was done as described for *Mt*AhpC.

Example 2

Measurement of NAD(P)H consumption

*Mt*MycR assay

The activity of AhpC with MycSH and MycR was tested in a system that contained 450 µM NADH or NADPH respectively, 0.2 µM - 20 µM *Mt*MycR, 0 µM - 20 µM MycSH, 0.2 µM - 20 µM *Mt*AhpC, 73 µM *t*-bOOH in 50 mM HEPES, 1 mM EDTA pH 7.4 in a final volume of 500 µl. The decrease in $A_{340}$ was monitored over time.

*Mt*AhpD/*Mt*AhpC assay

The specific activity of *Mt*AhpC with *Mt*AhpD was obtained by measuring initial velocities monitoring the NADH oxidation at 340 nm at 25°C. The test system contained 450 µM NADH, 200 nM *Mt*AhpD, 0.4 U bovine liponamide dehydrogenase (Sigma, Taufenkirchen, Germany), 50 µM liponamide, 0.5 µM - 10 µM *Mt*AhpC, 73 µM *t*-bOOH in 50 mM HEPES, 1 mM EDTA pH 7,4 in a final volume of 500 µl. The reaction was preincubated for 15 min at 25°C and started by addition of the peroxide. For the AhpD-dependent kinetic studies of *Mt*AhpC the conditions were nearly the same as for the measurement of the activity using 5 µM *Mt*AhpC and fixed *Mt*AhpD concentrations. A range between 5 µM - 65 µM *t*-bOOH was used for the calculation of the kinetic pattern.

*Mt*AhpC and *Mt*TPx assay

The specific activities of *Mt*AhpC with *Mt*TrxC and *Mt*TPx with *Mt*TrxB or *Mt*TrxC respectively were obtained by measuring initial velocities monitoring the NADPH oxidation at 340 nm at 25°C. The test system contained 450 µM NADPH, 10 µM *Mt*TR, 10 µM *Mt*TrxB or *Mt*TrxC, 0.5 µM - 15 µM *Mt*AhpC or 0.2 µM - 5 µM *Mt*TPx, 100 nM bovine catalase (Roche, Mannheim, Germany), 73 µM *t*-bOOH in 50 mM HEPES, 1 mM EDTA pH 7.4 in a final volume of 500 µl. The reaction was preincubated for 15 min at 25°C and started by addition of the peroxide. *Mt*TR and *Mt*Trx alone showed NADPH consumption. It was reported, that thioredoxin reductases produce $H_2O_2$. In this test system this would start the redox cascade. To reduce this basal acitivity to a minimum, catalase was added. For the kinetic studies of *Mt*AhpC and *Mt*TPx the conditions were nearly the same as for the measurement of the activities using 5 µM of the peroxidase and fixed thioredoxin concentrations. A range between 5 µM - 65 µM *t*-bOOH was used for the calculation of the kinetic pattern. Specificity for $H_2O_2$, cumene hydroperoxide, linoleic acid hydroperoxide and phosphatidyl cholin hydroperoxide, both prepared from soybean lipoxygenase-catalyzed oxidation of the corresponding lipids, were investigated accordingly.

Example 3

Steady-state-kinetics

Substrate turnover by peroxidases was monitored continuously by NAD(P)H consumption in a coupled test system. The data was analyzed according to Dalziel, (1957), *Acta Chem. Scand.* 11, 1706-1723. Results are presented in terms of the general Dalziel-equation for two substrate reactions, in which the term □$_{1,2}$/ [A] [B] is zero ( [$E_0$] /v = $\Phi_0$+ □$_1$/ [A] + □$_2$/ [B] + □$_{1,2}$/[A] [B]; see table 1). In this equation [$E_0$] means total enzyme molarity, [A] and [B] are concentrations of the oxidizing, and reducing substrate, respectively. The empirical coefficients $\Phi_0$, □$_1$, □$_2$ and □$_{1,2}$ are functional characteristics of the particular enzyme. The reciprocal net forward rate constant $k'_1$ by definition equals □$_1$ and □$_2$ describes a reductant-dependent step, thus must be the reciprocal $k'_2$. By analogy, $\Phi_0$ is the reciprocal $k_{cat}$, which can be any of the rate constants determining the a substrate-independent step.

Example 4

Gel permeation of native *Mt*TPx

*Mt*TPx was reduced by adding 200 mM DTT and chromatographed on a Superdex 75 column (Amersham Biosciences, Freiburg, Germany). protein molecular weight standards (Serva, Heidelberg, Germany) were used for column calibration. Eluted peroxidase was identified by activity measurements and SDS-PAGE. For size determination of oxidized *Mt*TPx, the enzyme was incubated with 300 µM t-bOOH at room temperature for 30 min. The molecular weight was then estimated by gel filtration chromatography as described above.

**Summary of examples**

Figure 1 shows the oxidative activation of *Mt*AhpC and *Mt*AhpD by *t*-bOOH. *Mt*AhpC activity with *Mt*AhpD as cosubstrate was tested in the conventional test system (*11*). Trace A: The reaction was preincubated for 15 min at 25°C and started by the addition of *t*-bOOH. Trace B: The reaction except *Mt*AhpC, *Mt*AhpD and *t*-bOOH was preincubated for 15 min at 25°C. MtAhpC, MtAhpD and the hydroperoxide were preincubated together for 2 min at 25°C and the reaction was started by the addition of this mixture.

Figure 2 shows steady-state-kinetic analysis of *Mt*TPx with *Mt*TrxB or *Mt*TrxC as cosubstrates. Substrate turnover by *Mt*TPx was monitored continuously by NADPH consumption in a coupled test system at 25°C and pH 7.4 with *t*-butyl hydroperoxide as substrate and *Mt*TrxB as cosubstrate. Three sets of data were analyzed according to Dalziel (Dalziel, (1957), *supra*). Results are presented in terms of the general Dalziel-equation for two-substrate reaction ([$E_o$]/v = $\Phi_0$+ □$_1$/[ROOH] + □$_2$/[reductant]; see table 1).

Figures 2A is an example of a primary Dalziel plot of the *Mt*TPx/*Mt*TrxB system showing enzyme-normalized inverse initial velocities ([$E_o$]/v) in dependence of reciprocal hydroperoxide concentration at different *Mt*TrxB concentrations that were kept constant by continuous regeneration. The slopes correspond to □$_1$.

Figure 2B is an example of a secondary Dalziel plot: The ordinate intercepts of (A), i. e., the apparent maximum velocities at [A] = ∞, are plotted against 1/[*Mt*TrxB] for evaluation of Dalziel coefficient $\Phi_2$ (slope), limiting $K_{MMtTrxB}$

(abscissa intercept is $1/K_{MMtTrxB}$), and real maximum velocities (ordinate intercept is $\Phi_0 = [E_0]/V_{max}$). Primary data of three independent analyzes, as shown in (A), were used to obtain means and standard deviations.

Figure 2C is an example of a primary Dalziel plot of the *Mt*TPx/*Mt*TrxC system showing enzyme-normalized inverse initial velocities ($[E_o]/v$) in dependence of reciprocal hydroperoxide concentration at different *Mt*TrxC concentrations that were kept constant by continuous regeneration.

Table 1 summarizes the kinetic constants of MtAhpC and MtTPx. Rate constants were derived from steady-state analysis as shown in Figure 2; the empirical Dalziel coefficients $\Phi_0$, $\Phi_1$ and $\Phi_2$ correspond to the reciprocal values of $k_{cat}$, $k'_1$ and $k'_2$, the latter two being net forward rate constants for the reaction of the enzymes with *t*-bOOH and the reductants, respectively.

Table 1

| enzyme | donor substrate $[s^{-1}]$ | $k_{cat}$ $[s^{-1}]$ | $k'_1$ $[M^{-1}s^{-1}]$ | $k'_2$ $[M^{-1}s^{-1}]$ | $K_M$ ROOH [µM] | $K_M$ donor substrate [µM] |
|---|---|---|---|---|---|---|
| *Mt*AhpC | *Mt*AhpD | 0.63 ± 0.94 | $0.95 \times 10^4 \pm 0.08$ | $2.7 \times 10^6 \pm 14.28$ | 65.6 | 0.23 |
| *Mt*AhpC | *Mt*TrxC | 0.13 ± 1.21 | $2.3 \times 10^4 \pm 0.07$ | $2.5 \times 10^4 \pm 0.12$ | 5.64 | 5.10 |
| *Mt*TPx | *Mt*TrxB | 0.66 ± 1.12 | $3.4 \times 10^5 \pm 0.48$ | $4.6 \times 10^4 \pm 1.35$ | 1.95 | 14.46 |
| *Mt*TPx | *Mt*TrxC | 11.11 ± 0.99 | $0.9 \times 10^5 \pm 0.22$ | $5.8 \times 10^4 \pm 0.22$ | 120. 68 | 184.4 |

Table 2 summarizes the specificity of MtAhpC and MtTPx for hydroperoxide substrates. The activities with t-butyl hydroperoxide (*t*-bOOH) were set to 100 %. LOOH, PCOOH and COOH mean linoleic acid hydroperoxide, phosphatidyl cholin hydroperoxide and cumene hydroperoxide, respectively. * The activities with $H_2O_2$ were difficult to be estimated due to the basal activity of TR/Trx system.

Table 2

| enzyme | donor substrate | *t*-bOOH | LOOH | PCOOH | $H_2O_2$ | COOH |
|---|---|---|---|---|---|---|
| *Mt*AhpC | TrxC | 100 | 30 | 0 | 140* | 60 |
| *Mt*TPx | TrxB | 100 | 0 | 6 | 150* | 54 |

**Reference list**

1. A. Pablos-Mendez et al., *N. Engl. J. Med.* 338, 1641-1649 (1998).
2. D. R. Sherman et al., *Science* 272, 1641-1643 (1996).
3. Y. Zhang, B. Heym, B. Allen, D. Young, S. Cole, *Nature* 358, 591-593 (1992).
4. D. R. Sherman, K. Mdluli, M. J. Hickey, C. E. Barry, III, C. K. Stover, *Biofactors* 10, 211-217 (1999).
5. B. Hofmann, H. J. Hecht, L. Flohe, *Biol. Chem.* 383, 347-364 (2002).
6. Z. A. Wood, E. Schroeder, H. J. Robin, L. B. Poole, *Trends Biochem. Sci.* 28, 32-40 (2003).
7. S. T. Cole et al., *Nature* 393, 537-544 (1998).
8. P. J. Hillas, F. S. del Alba, J. Oyarzabal, A. Wilks, P. R. Ortiz de Montellano, *J. Biol. Chem.* 275, 18801-18809 (2000).
9. B. Vergauwen, F. Pauwels, M. Vaneechoutte, J. J. van Beeumen, *Arch. Physiol. Biochem.* 113, B22 (2001).
10. R. Bryk, C. D. Lima, H. Erdjument-Bromage, P. Tempst, C. Nathan, *Science* 295, 1073-1077 (2002).
11. C. M. Nunn, S. Djordjevic, P. J. Hillas, C. R. Nishida, P. R. Ortiz de Montellano, *J. Biol. Chem.* 277, 20033-20040 (2002).
12. R. Chauhan and S. C. Mande, *Biochem. J.* 354, 209-215 (2001).
13. Z. A. Wood, L. B. Poole, R. R. Hantgan, P. A. Karplus, *Biochemistry* 41, 5493-5504 (2002).
14. L. M. Baker and L. B. Poole, *J. Biol. Chem.* 278, 9203-9211 (2003).
15. P. R. Jungblut et al., *Mol. Microbiol.* 33, 1103-1117 (1999).
16. H. J. Mollenkopf et al., *Electrophoresis* 20, 2172-2180 (1999).
17. S. Nagai, H. G. Wiker, M. Harboe, M. Kinomoto, *Infect. Immun.* 59, 372-382 (1991).

18. M. G. Sonnenberg and J. T. Belisle, *Infect. Immun.* 65, 4515-4524 (1997).
19. K. Weldingh et al., *Infect. Immun.* 66, 3492-3500 (1998).
20. I. Rosenkrands et al., *Electrophoresis* 21, 935-948 (2000).
21. I. Rosenkrands et al., *Electrophoresis* 21, 3740-3756 (2000).
22. L. Flohé and R. Brigelius-Flohé, in *Selenium. Its Molecular Biology and Role in Human Health,* D. L. Hatfield, Ed. (Kluwer Academic Publishers, Boston/Dordrecht/London, 2001).
22. K. Dalziel, *Acta Chem*. Scand. 11, 1706-1723 (1957).
23. M. D. Unson, G. L. Newton, C. Davis, R. C. Fahey, *J. Immunol. Methods* 214, 29-39 (1998).

## Claims

1. An enzyme-based assay for detecting inhibitors of the anti-oxidant defense system in *Mycobacterium* comprising

   (i) a reductant;
   (ii) a thioredoxin reductase or a peroxidase;
   (iii) a donor substrate capable of receiving a hydrogen atom from (ii); and
   (iv) a hydroperoxide,

   for determining the consumption of the reductant.

2. The assay of claim 1, wherein the thioredoxin reductase and/or the peroxidase and/or the donor substrate is an enzyme derived from *Mycobacterium*, preferably *Mycobacterium tuberculosis.*

3. The assay of claim 1 or 2, wherein the enzyme is expressed in heterologous host cells.

4. The assay of claim 3, wherein the host cells are selected from the group consisting of bacteria, yeasts, insect cells and mammalian cells, preferably cells of *Escherichia coli, Saccharomyces cerevisiae* and *Spodoptera frugiperda.*

5. The assay of any of the preceding claims, wherein the reductant is NADH, NADPH and/or FADH.

6. The assay of any of the preceding claims, wherein the peroxidase is a peroxiredoxin, preferably AhpC and/or TPx of *Mycobacterium,* more preferably of *Mycobacterium tuberculosis.*

7. The assay of any of the preceding claims, wherein the donor substrate is selected from the group consisting of glutathione and thioredoxin, preferably thioredoxin B (TrxB) and/or thioredoxin C (TrxC) of *Mycobacterium,* more preferably of *Mycobacterium tuberculosis.*

8. The assay of any of the preceding claims, wherein the hydroperoxide is selected from the group consisting of hydrogen peroxide, t-butyl hydroperoxide, cumene hydroperoxide, linoleic acid hydroperoxide, phosphatidyl choline hydroperoxide and any oxidant comprising a hydroperoxy group.

**9.** A method for manufacturing a pharmaceutically active compound for treating and/or preventing a *Mycobacterium* associated disease, comprising the steps of

(a) providing an enzyme-based assay of any of claims 1 to 8;
(b) providing a test sample comprising a potentially active compound;
(c) contacting (a) and (b);
(d) determining consumption of a reductant;
(e) recovering the active compound.

**10.** The method of claim 9, wherein the reductant is NADH, NADPH and/or FADH.

**11.** The method of claim 9 or 10, wherein the active compound is combined with suitable pharmaceutically acceptable fillers, excipients and/or additives.

**12.** The method of claim 11, wherein the pharmaceutically acceptable filler, excipients and/or additives are selected from the group consisting of physiological saline solution, demineralized water, stabilizers, proteinase inhibitors and gel formulations.

**13.** A use of the pharmaceutically active compound identified by the method of any of claims 9 to 12, for the preparation of a medicament for treating and/or preventing a *Mycobacterium* associated disease.

**14.** The use of claim 13, wherein the disease is tuberculosis.

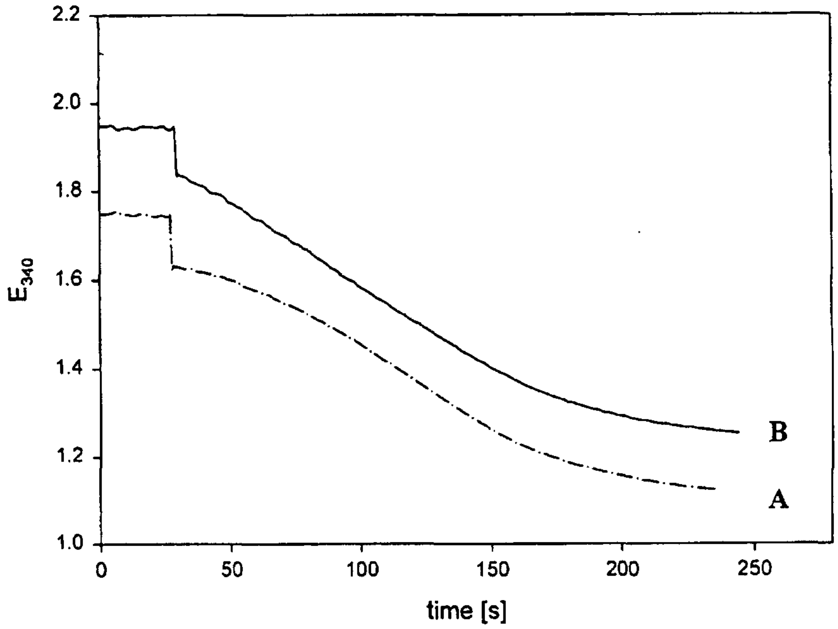


Figure 1

primary plot

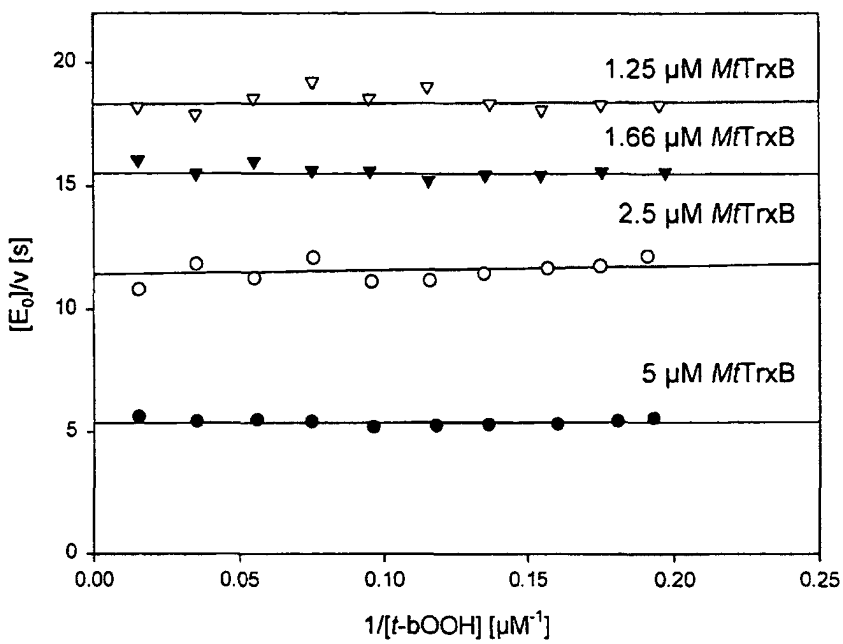


Figure 2A

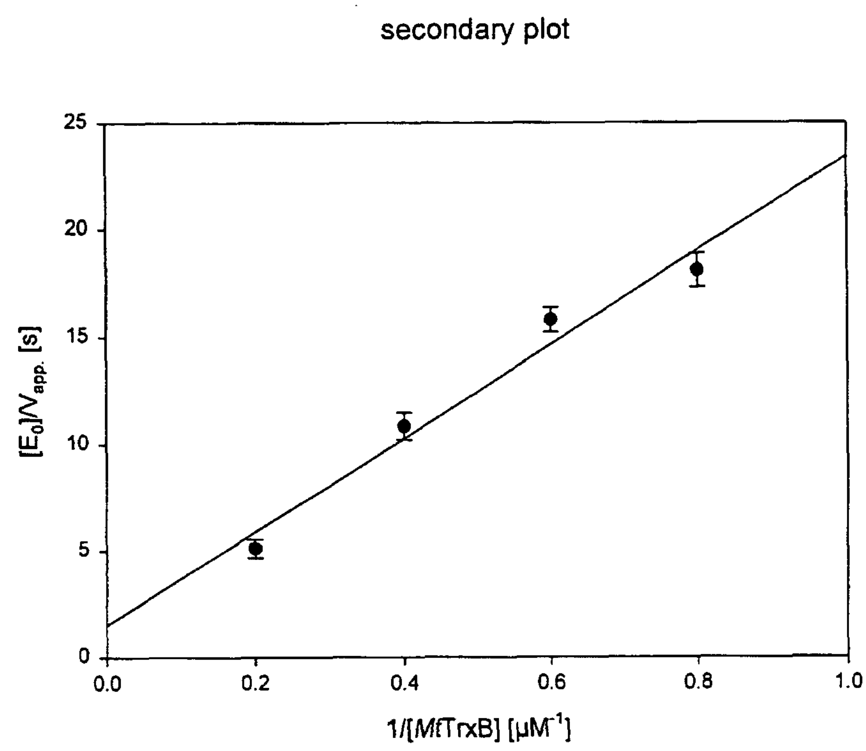
secondary plot
25
20
15
10
5
0
0.0
0.2
0.4
0.6
0.8
1.0
[E0]/Vapp. [s]
1/[MfTrxB] [µM-1]

Figure 2B

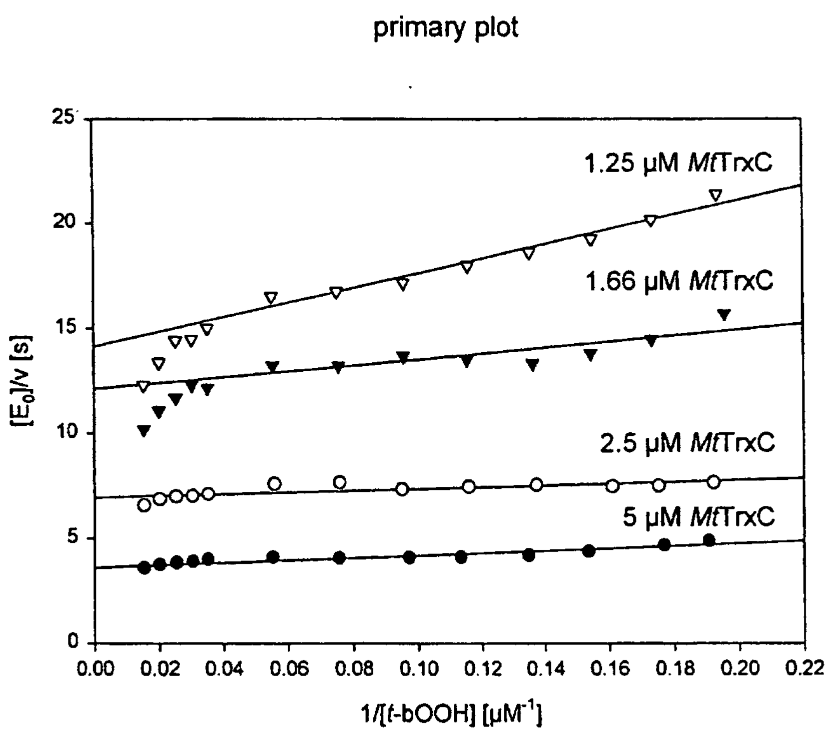
primary plot
1.25 µM MfTrxC
1.66 µM MfTrxC
2.5 µM MfTrxC
5 µM MfTrxC
[E0]/v [s]
1/[t-bOOH] [µM-1]
0
5
10
15
20
25
0.00
0.02
0.04
0.06
0.08
0.10
0.12
0.14
0.16
0.18
0.20
0.22

Figure 2C

European Patent Office

## PARTIAL EUROPEAN SEARCH REPORT

which under Rule 45 of the European Patent Convention shall be considered, for the purposes of subsequent proceedings, as the European search report

Application Number EP 03 01 2155

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 96 19578 A (COLLINS DESMOND MICHAEL ;WILSON THERESA (NZ); PASTORAL AGRIC RES I) 27 June 1996 (1996-06-27)<br>* the whole document *<br>* page 5, line 12 - line 25 *<br>* page 16, line 24 - line 32 *<br>* claims 19,20 *<br>--- | 1-6,8-12 | C12Q1/18<br>C12Q1/26<br>C12Q1/28 |
| D,X | HILLAS PATRICK J ET AL: "The AhpC and AhpD antioxidant defense system of Mycobacterium tuberculosis"<br>JOURNAL OF BIOLOGICAL CHEMISTRY, vol. 275, no. 25, 23 June 2000 (2000-06-23), pages 18801-18809, XP002260881<br>ISSN: 0021-9258<br>* the whole document *<br>* page 18803, left-hand column, line 47 - line 57 *<br>* page 18809, left-hand column, line 47 - line 57 *<br>* abstract *<br>---<br>-/-- | 1-6,8-12 | |

TECHNICAL FIELDS SEARCHED (Int.Cl.7)

C12Q

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| THE HAGUE | 11 November 2003 | Tuynman, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 03 01 2155

Claim(s) not searched:
13,14

Reason for the limitation of the search:

Present claims 13 and 14 relate to the use of compounds defined by reference to a desirable characteristic or property, namely compounds being identified according to a method used in claims 9-12.

The claims cover all compounds having this characteristic or property, whereas the application provides no support within the meaning of Article 84 EPC and no disclosure within the meaning of Article 83 EPC for only a very limited number of such compounds. In the present case, the claims so lack support, and the application so lacks disclosure, that a meaningful search over the whole of the claimed scope is impossible. Independent of the above reasoning, the claims also lack clarity (Article 84 EPC). An attempt is made to define the compound by reference to a result to be achieved. Again, this lack of clarity in the present case is such as to render a meaningful search over the whole of the claimed scope impossible. Consequently, the search has not been carried out for these claims.

European Patent Office

# PARTIAL EUROPEAN SEARCH REPORT

Application Number
EP 03 01 2155

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| Y | WO 03 004479 A (CANCER RES TECHNOLOGY LTD ;POOLE TRACEY DAWN (GB); WESTWELL ANDREW) 16 January 2003 (2003-01-16) * page 91, line 14 - line 29 * * page 96, line 11 - page 97, line 3 * * page 102, line 11 - line 13 * * page 141, line 5 - page 142, line 9 * --- | 1-12 | |
| Y | ZHANG ZHOUPENG ET AL: "Reduction of peroxides and dinitrobenzenes by Mycobactgerium tuberculosis thioredoxin and thioredoxin reductase" ARCHIVES OF BIOCHEMISTRY AND BIOPHYSICS, vol. 363, no. 1, 1 March 1999 (1999-03-01), pages 19-26, XP002260882 ISSN: 0003-9861 * the whole document * --- | 1-12 | TECHNICAL FIELDS SEARCHED (Int.Cl.7) |
| D,Y | NUNN CHRISTINE M ET AL: "The crystal structure of Mycobacterium tuberculosis alkylhydroperoxidase AhpD, a potential target for antitubercular drug design" JOURNAL OF BIOLOGICAL CHEMISTRY, vol. 277, no. 22, 31 May 2002 (2002-05-31), pages 20033-20040, XP002260884 ISSN: 0021-9258 * abstract * --- -/-- | 1-6,8-12 | |

EPO FORM 1503 03.82 (P04C10)

European Patent Office

# PARTIAL EUROPEAN SEARCH REPORT

Application Number
EP 03 01 2155

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| Y | KOSHKIN ALEKSEY ET AL: "The mechanism of Mycobacterium tuberculosis alkylhydroperoxidase AhpD as defined by mutagenesis, crystallography, and kinetics" J. BIOL. CHEM.;JOURNAL OF BIOLOGICAL CHEMISTRY AUG 8 2003, vol. 278, no. 32, 21 March 2003 (2003-03-21), pages 29502-29508, XP002260883 * abstract * * page 29503, right-hand column, line 45 - line 63 * ----- | 1-6,8-12 | |
| | | | TECHNICAL FIELDS SEARCHED (Int.Cl.7) |

EPO FORM 1503 03.82 (P04C10)

## ANNEX TO THE EUROPEAN SEARCH REPORT ON EUROPEAN PATENT APPLICATION NO. EP 03 01 2155

This annex lists the patent family membersrelating to the patent documents cited in the above-mentioned European search report.
The members are as contained in the European Patent Office EDP file on
The European Patent Office is in no way liable for these particulars which are merely given for the purpose of information.

11-11-2003

| Patent document cited in search report | | Publication date | Patent family member(s) | | | Publication date |
|---|---|---|---|---|---|---|
| WO 9619578 | A | 27-06-1996 | AU | 708456 | B2 | 05-08-1999 |
| | | | AU | 4318596 | A | 10-07-1996 |
| | | | CA | 2210378 | A1 | 27-06-1996 |
| | | | EP | 0805863 | A2 | 12-11-1997 |
| | | | WO | 9619578 | A2 | 27-06-1996 |
| WO 03004479 | A | 16-01-2003 | WO | 03004479 | A1 | 16-01-2003 |

EPO FORM P0459

For more details about this annex : see Official Journal of the European Patent Office, No. 12/82

SEQUENCE LISTING

<110> Flohé, Leopold

<120> Enzyme-based assay, method for treating Mycobacterium associated diseases and uses thereof

<130> Fl03A01/P-DE

<160> 16

<170> PatentIn version 3.1

<210> 1

<211> 36

<212> DNA

<213> Artificial Sequence

<220> Feature

<221> misc_feature

<222> (1)..(6)

<223> n = a, t, c, or g

<220>

<221> source

<223> Description of artificial sequence: primer

<400> 1
nnnnnnctgc agatggaaac gtacgacatc gcgatc 36

<210> 2

<211> 54

<212> DNA

<213> Artificial Sequence

<220> Feature

<221> misc_feature

<222> (1)..(6)

<223> n = a, t, c, or g

<220>

<221> source

<223> Description of artificial sequence: primer

<400> 2
nnnnnnaagc tttcagtgat ggtgatggtg atgacgcagg ccaagcagcg cgtt 54

<210> 3

<211> 33

<212> DNA

<213> Artificial Sequence

<220>

<221> source

<223> Description of artificial sequence: primer

<400> 3
ggcgcgccat atgccactgc taaccattgg cga 33

<210> 4

<211> 53

<212> DNA

<213> Artificial Sequence

<220>

<221> source

<223> Description of artificial sequence: primer

<400> 4
ccggcgggat ccttagtggt ggtggtggtg gtgggccgaa gccttgagga gtt 53

<210> 5

<211> 38

<212> DNA

<213> Artificial Sequence

<220>

<221> source

<223> Description of artificial sequence: primer

<210> 9

<211> 30

<212> DNA

<213> Artificial Sequence

<220>

<221> source

<223> Description of artificial sequence: primer

<400> 9
cccccccata tgctcgagaa ggccccccag 30

<210> 10

<211> 57

<212> DNA

<213> Artificial Sequence

<220>

<221> source

<223> Description of artificial sequence: primer

<400> 10
ccccccgaat tctcagtgat ggtgatggtg atgggatgaa gtctttgttc cagggcc 57

<210> 11

<211> 54

<212> DNA

<213> Artificial Sequence

<220>

<221> source

<223> Description of artificial sequence: primer

<400> 11
ccccccgaat tctcagtgat ggtgatggtg atgggcttgt tgggctcgcc cgtt 54

<210> 12

<211> 54

<400> 5
ccccccccca tatgagtata gaaaagctca aggccgcg 38

<210> 6

<211> 38

<212> DNA

<213> Artificial Sequence

<220>

<221> source

<223> Description of artificial sequence: primer

<400> 6
cccccccaa gcttttagct tgggcttagt gcctcgat 38

<210> 7

<211> 33

<212> DNA

<213> Artificial Sequence

<220>

<221> source

<223> Description of artificial sequence: primer

<400> 7
cccccccata tggtgaccac tcgagacctc acg 33

<210> 8

<211> 36

<212> DNA

<213> Artificial Sequence

<220>

<221> source

<223> Description of artificial sequence: primer

<400> 8
cccccccata tggtgactac ccgagacctc actgcc 36

<212> DNA

<213> Artificial Sequence

<220>

<221> source

<223> Description of artificial sequence: primer

<400> 12
cccccccgaat tcctagtggt ggtggtggtg gtggtcgaac tgaggcggct cggt 54

<210> 13

<211> 53

<212> DNA

<213> Artificial Sequence

<220>

<221> source

<223> Description of artificial sequence: primer

<400> 13
ccccccccca tatgcatcac catcaccatc acaccgattc cgagaagtcc gcc 53

<210> 14

<211> 51

<212> DNA

<213> Artificial Sequence

<220>

<221> source

<223> Description of artificial sequence: primer

<400> 14
cccccccata tgcatcacca tcaccatcac gcacagataa ccctgcgagg a 51

<210> 15

<211> 38

<212> DNA

<213> Artificial Sequence

<220>

<221> source

<223> Description of artificial sequence: primer

<400> 15
cccccccaa gcttctagtt gaggttggga accacgtc 38

<210> 16

<211> 30

<212> DNA

<213> Artificial Sequence

<220>

<221> source

<223> Description of artificial sequence: primer

<400> 16
cccccaagc ttctaggcgc ccagcgcggc 30